# EUROPEAN PATENT SPECIFICATION

(11) **EP 2 332 519 B1**
(45) Date of publication and mention of the grant of the patent: **28.11.2018**
(21) Application number: 09811451.5
(22) Date of filing: 28.08.2009
(51) Int. Cl.: A61Q 1/02, A61Q 1/10, A61Q 1/12, A61K 8/02, A61K 8/31, A61K 8/37, A61K 8/73, A61K 8/92, C08L 1/02

(54) **POWDER COSMETIC MATERIAL**
KOSMETISCHES PULVERMATERIAL
MATÉRIAU COSMÉTIQUE EN POUDRE

(30) Priority: 03.09.2008 WO PCT/JP2008/065810
(43) Date of publication of application: 15.06.2011
(73) Proprietor: Ohken Co., Ltd., Chiyoda-ku Tokyo 101-0032 (JP); National Institute of Advanced Industrial Science and Technology, Tokyo 100-8921 (JP); Hyogo Prefecture, Kobe-shi, Hyogo 650-8567 (JP)
(72) Inventor: MURATA, Togo, Chiyoda-ku Tokyo 101-0032 (JP); MURATA, Tetsuo, Chiyoda-ku Tokyo 101-0032 (JP); ENDO, Takashi, Higashi-hiroshima-shi Hiroshima 739-0046 (JP); NAGATANI, Asahiro, Kobe-shi Hyogo 654-0037 (JP)
(74) Representative: Michalski Hüttermann & Partner Patentanwälte mbB
(86) International application number: PCT/JP2009/065056
(87) International publication number: WO 2010/026925

(56) References cited:
- DE-A1- 1 940 953
- JP-A- 58 072 512
- JP-A- 2000 309 508
- JP-A- 2000 327 519
- JP-A- 2002 275 034
- JP-A- 2004 230 719
- JP-A- 2004 231 796
- JP-A- 2007 269 697
- JP-A- 2007 314 454
- JP-B2- 3 787 598
- US-A- 2 709 045
- US-A- 5 024 831
- US-A1- 2004 156 811

## Description

### Technical Field

The present invention relates to powder cosmetics. More particularly, it relates to powder cosmetics into which a larger amount of oily components can be compounded than in the conventional powder cosmetics.

### Background Art

Powder cosmetics containing powder components, such as powder foundation and eye shadow, are placed in a major category in the field of cosmetics.

These powder cosmetics can be produced by dispersing powder components such as an extender, pigment, homogeneously into an oily component as binder to give a powder mixture, and molding the mixture placed on a metal plate by press packing.

Among the powder components used in the above-mentioned powder cosmetics, naturally occurring thin sheet-like mica flake, sericite, talc, or their surface-processed products have been employed as an extender because they are excellent in extensibility and adhesiveness to the skin. In addition, organic powder including really spherical nylon powder has been used together with the above extender to improve the smoothness or touch of the extender on the skin. Further, white pigment such as titanium dioxide, coloring pigment such as iron oxide red, and pearlescent pigment such as mica titanium, have been used as pigments.

In preparing powder cosmetics with the above powder components, the total amount of the oily components blended into the powder cosmetics has been limited approximately to the range of 15 mass % or less, though a little difference is recognized depending on the kind of powder component. The reason is that the excessive amount of the oily component over the above-mentioned range yields aggregation in the powder to give a product far from a powder cosmetic, and further it forms caking on the surface of fillers (a phenomenon that the surface becomes so hard as to make its removal with a powder puff or sponge difficult), causing a problem to make the use as powder cosmetic difficult, though there is a favorable tendency to increase a moisture feeling and fitting to the skin with increase in the amount of the oily component.

### Disclosure of Invention

### Problem to be Solved by the Invention

As mentioned above, since the moisture feeling and fitting to the skin increase with increasing amount of oily component, there is a need to compound a large amount of the oily component in a powder cosmetic. Thus, there has been a growing demand for a powder component for cosmetics, which hardly causes aggregation even if a large amount of oily component is added, and for a powder cosmetic utilizing such a cosmetic powder, with which almost no caking of the powder component is generated on the surface of a pressed product despite a large amount of oily component comprised therein. The object of the present invention is to provide such a cosmetic.

### Means for Solving the Problems

In order to solve the above problems, the present inventors worked to search a powder component which allows powder cosmetics to contain a larger amount of oily component from a wide variety of organic and inorganic compounds. As a result, the inventors found that the blending of tabular oblate cellulose particles as powder component into a cosmetic leads to solve the above problems. Thus, the present invention was completed.

The invention is defined by the independent claims. The dependent claims describe preferred embodiments of the invention.

Namely, the present invention provides powder cosmetics which comprise oblate cellulose particles and an oily component, wherein the content of the oily component is 15 to 40% by mass.

### Effect of the Invention

The present invention provides excellent powder cosmetics, in which no aggregation of powder occurs, the character of powdery feel of the powder itself is kept, and no caking occurs in a pressed product, even when a large amount of oily component is blended. In addition, the powder cosmetic can favorably be removed with a sponge, and when applied to the skin, it results in a long lasting make-up which naturally blends with the skin without unevenness, kink, somber color, and coming-off.

Further, in the powder cosmetics of the present invention, the above characteristics are maintained favorably even if the content of titanium dioxide is increased in addition to the oily component, while avoiding unnatural whiteness and thickness when applied.

Thus, the powder cosmetics of the present invention can favorably be used as powder foundation, eye shadow, face powder.

### Best Mode for Carrying Out the Invention

The oblate cellulose particles used in the powder cosmetics of the present invention are not particularly limited, as far as the cellulose particles are oblate in shape. For example, cellulose particles having the average particle size of 1 - 100µm, preferably 1 - 50µm, and the average thickness of 0.1 - 10µm, preferably 1 - 3µm, may be used. Among the above particles, those having the oblateness of 3 - 20, particularly 5 - 15, may preferably be used as the oblate cellulose particles in the powder cosmetics of the present invention.

As mentioned below, when a grinding aid and an N-acyl amino acid as a hydrophobizing agent are used in combination in producing oblate cellulose particles, hydrophobicity is given to the oblate cellulose particles with decrease of the average thickness and increase of the oblateness without changing the average particle size. In this case, the resulting oblate cellulose particles have the average particle size of 1 - 100µm, preferably 1 - 50µm, the average thickness of 0.01 - 5µm, preferably 0.01 - 2µm, and the oblateness of 50 - 200. The oblate cellulose particles can favorably be used in the powder cosmetics of the present invention. Further, the oblate cellulose particles of this size are new materials which can be used in the conventional cosmetics other than the powder cosmetics. When the oblate cellulose particles of this size are used in the conventional cosmetics, they may be used merely in place of the conventional extender or cellulose materials.

In this context, the average particle size means the average value of the width and length of the oblate cellulose particles on a particle size distribution measuring apparatus (the values of particle size in 50% of the integrated volume), which are determined in an aqueous suspension by means of, for example, an apparatus based on laser diffraction scattering. The average thickness means the average value obtained by choosing plural particles equivalent to the average particle size obtained as above on an electron microscope such as scanning electron microscope and measuring their thickness, followed by averaging. The oblateness means the value of average particle size/average thickness obtained as above.

The above oblate cellulose particles may be produced according to a known method as described in, for example, Japanese Patent 2,560,235, and Japanese Patent 3,787,598, or its modified method.

Specifically, the oblate cellulose particles may be produced according to the following method (a) or (b).
(a) A method which comprises mixing a cellulosic material with at least one of grinding aids selected from the group consisting of water, fatty acids, synthetic polymers and organic solvents, followed by mechanical grinding.
(b) A method which comprises freezing a cellulosic material at a low temperature, followed by mechanical grinding.

Among the above methods, the method (a) may be carried out basically according to the description of Japanese Patent 2,560,235, paragraphs [0005] to [0009] and Japanese Patent 3, 787, 598, paragraphs [0020] to [0041].

There is no particular limitation in cellulosic materials used as starting materials in the above method (a). For example, fibrous or powdered wood flour or wood pulp derived from wood, fibrous or powdered cotton or linter derived from raw cotton, or their purified fibrous or powdered cellulosic materials are preferably used, and in particular, acidically hydrolyzed and purified cellulosic materials are preferred. In order to practically use the physiological activity of the raw materials, wood flour or bamboo flour may be used as starting materials. The cellulosic materials contain around 5 - 10% by mass (hereinafter merely referred to as "%") of adsorbed moisture in an air-dried state, which may be further dried before the grinding process under hot air, in vacuum, or under reduced pressure to change the amount of adsorbed moisture, so that the size of oblate cellulose particles obtained after the grinding process can be controlled to some degree.

The grinding aids used in the method (a) include water, fatty acids, synthetic polymers and organic solvents. They may be used alone or in combination of two or more.

The water used as a grinding aid includes distilled water, ion-exchange water, reverse osmotic water. The water may be added so as to be around 1 - 20%, preferably 2 - 9%, to the cellulosic material.

The fatty acids include saturated fatty acids such as stearic acid, saturated fatty acid derivatives thereof such as zinc salts, unsaturated fatty acids such as oleic acid, and unsaturated fatty acid derivatives thereof such as sodium salts. Among these fatty acids, saturated fatty acids are preferred, and in particular stearic acid is preferred. The fatty acids may be added so as to be around 0.1 - 100%, preferably 1 - 20%, to the cellulosic material.

The synthetic polymer includes polyalcohols such as polyvinyl alcohol, polyethers such as polyethylene glycol, polyolefins such as polyethylene, polyamides. Among these synthetic polymers, polyethylene glycol is preferred. The synthetic polymers may be added so as to be around 0.1 - 100%, preferably 5 - 30%, to the cellulosic material.

The organic solvent includes alkanes such as hexane, alcohols such as ethanol, ketones such as acetone, ethers such as tetrahydrofuran, aromatic hydrocarbons such as toluene. The organic solvent may be added so as to be around 1 - 100%, preferably 20 - 50%, to the cellulosic material.

In addition, by adding a hydrophobizing agent selected from the group consisting of N-acyl amino acids and reactive silicones together with the aforementioned grinding aid, it is possible to give the oblate cellulose particles hydrophobicity and affinity for the oil to be used. The hydrophobizing agents may be used alone or in combination of two or more.

The N-acyl amino acids used as hydrophobizing agent include N^{ε}-lauroyllysine, N^{α}-hexanoyllysine, N^{α}-oleyllysine, N^{α}-lauroyllysine, N^{α}-myristonoyllysine, N^{α}-palmitoyllysine, N^{α}-steanoyllysine, N^{ε}-hexanoyllysine, N^{ε}-oleyllysine, N^{ε}-myristonoyllysine, N^{ε}-palmitoyllysine, N^{ε}-steanoyllysine, Among these N-acryl amino acids, N^{ε}-lauroyllysine is preferred, and in particular N^{ε}-lauroyl-L-lysine preferred.

The reactive silicone includes triethoxysilylethylpolydimethylsiloxyethyldimethicone, triethoxysilylethyl-polydimethylsiloxyethylhexyldimethicone. Among these reactive silicones, triethoxysilylethylpolydimethylsiloxyethyldimethicone is preferred.

The hydrophobizing agent may be added so as to be around 0.1 - 10%, preferably 0.5 - 3%, to the cellulosic material.

In the mechanical grinding process conducted in the method (a), pressure or shearing press for powdering has to be applied continuously for a certain period of time. In this situation, it is appropriate in a grinding process to use a mill such as vibrating ball mill, rotating ball mill, planetary ball mill, roll mill, disc mill, high-speed mixer with high speed rotary blade, homo-mixer rather than using a crusher mainly based on cutting mechanism for samples, such as cutter mill or Wiley mill, and particularly, a planetary ball mill is preferable. The grinding energy applied for the grinding process is appropriately 3 - 20G, preferably 5 - 15G.

Preferred embodiment for obtaining oblate cellulose particles in the above-described method (a) is shown as follows.

Purified cellulose powder derived from wood pulp is dried at 30 - 50°C under reduced pressure to remove adsorbed moisture well to 0.1% or less. The resulting cellulose powder is added together with grinding zirconia balls into a tightly-closable grinding zirconia vessel, and a grinding aid and if necessary a hydrophobizing agent are further added at any one of the following steps (i) - (iii). Thereafter, the above grinding vessel is placed on a planetary ball mill and subjected to grinding in a cycle of grinding rotation for 5 - 15 minutes and intermission for 5 - 15 minutes; this cycle is repeated 12 - 72 times continuously. In this operation, the rotational frequency is set at 100 - 250 rpm.

### (Grinding aid and method of addition thereof)

(i) Stearic acid is added so as to be 1 - 20% to the cellulose powder.
(ii) N^{ε}-lauroyllysine is added so as to be 0.5 - 3%, and water is added so as to be 2 - 9% to the cellulose powder.
(iii) Triethoxysilylethylpolydimethylsiloxyethyldimethicone is added so as to be 0.5 - 3%, and stearic acid is added so as to be 1 - 20% to the cellulose powder.

On the other hand, the above method (b) can be carried out according to a known freeze-grinding process without any limitation. Specifically, the method (b) may be carried out by freezing the cellulosic material used in the above method (a) at -5°C or lower, preferably -10 to -30°C, followed by applying to the same grinding apparatus as in the above method (a).

The oblate cellulose particles produced in the above methods may be used as such in the powder cosmetics of the present invention, and may further be subjected to a known drying tool such as air drying, hot air drying, vacuum drying, drying under reduced pressure, and so on to remove the grinding aid before using.

Thus resulting oblate cellulose particles as mentioned above are of thinner plates than the conventional extender such as talc and sericite, and have excellent properties in transparency, soft touch, extensibility, natural luster.

In particular, in the oblate cellulose particles produced with a grinding aid and a hydrophobizing agent, hydrophobicity and affinity for an oil used are improved in addition to the above properties.

The amount of oblate cellulose particles to be compounded in the cosmetics of the present invention is 5 - 90%, preferably 10 - 90%, in particular 15 - 70%.

On the other hand, the oily component for use in the powder cosmetics of the present invention is not particularly limited and includes those being liquid at room temperature, for example, hydrocarbon such as liquid paraffin, silicone oil such as KF99-1 (Shin-Etsu Chemical Co., Ltd.), ester oils such as octyldodecyl oleate (OOD; Shin-ei Chemical Co., Ltd.), fats and oils, higher fatty acids, higher alcohols; vaseline (paste state at room temperature); and cetanol (solid at room temperature). These oily components may be used in combination. Particularly, in the powder cosmetics of the present invention, the oily component being liquid at room temperature is preferably used in combination with paste-state one and/or solid one, because the resulting powder cosmetic can tighten slightly firm and the powder scraped off from the surface with a sponge becomes relatively small, thereby attaining the greater usability of the cosmetic. The content of the oily component to be blended in the powder cosmetics of the present invention is 15 - 40%, preferably 15 - 35%.

The powder cosmetics of the present invention comprise essentially oblate cellulose particles and an oily component, and otherwise they may be produced in the same manner as in the conventional cosmetics. Specifically, in the conventional process for producing cosmetics containing powder components and an oily component, a part or all of the powders may be replaced with the oblate cellulose particles.

In addition to the above-mentioned essential components, the powder cosmetics of the present invention may contain the following components used in the conventional cosmetics, as long as they do not hinder the effect of the invention: powder component including inorganic powder such as mica, talc, and sericite, organic powder or surface-processed products thereof such as silk powder, white pigments such as titanium dioxide, coloring pigments such as iron oxide red, and pearlescent pigments such as mica titanium; surface-activating component; moisturizing component; UV absorbing component; antiseptic component; beauty component; fragrance.

When oblate cellulose particles are used as a part or all of the powder components in the powder cosmetics of the present invention, it is possible to compound a larger amount of oily components than in the conventional powder cosmetics. In the context of the present application, the powder cosmetic comprising 15 - 40%, preferably 15 - 35% oily component is referred to as "oil-rich cosmetic".

In producing the above powder cosmetics comprising a large amount of oily component (oil-rich cosmetic), the oily component may be added to the cosmetic after molding in order to increase the content of the oily component, for example, according to the description of JP 7/55892B (1995).

Specifically, in order to increase the content of the oily component by adding the oily component to the cosmetic after molding, a powder cosmetic containing 5 - 20% oily component is press-molded in a metallic mold, and then a desired amount of oily component is dropped or sprayed onto the surface of the powder cosmetic so that the oily component is absorbed into the cosmetic without damaging the surface.

Thus resulting powder cosmetics of the present invention are capable of comprising a large amount of oily component as compared with the conventional cosmetics, and accordingly, another oily component which is particularly beneficial for the skin, such as squalane, may be contained besides the basic material oily component. Furthermore, even though the same amount of the oily component as that of the conventional powder cosmetics is blended, the resulting powder cosmetic can give powdery feel with less oiliness.

In the powder cosmetics of the present invention, a larger amount of oily component can be compounded than in the conventional cosmetics, and further 15 - 30%, preferably 16 - 24% titanium dioxide may be added. In the conventional powder cosmetics, this amount of titanium dioxide causes unnatural whiteness and thickness when applied. On the other hand, the powder cosmetics of the present invention are excellent in terms of overcoming such problems.

The cosmetics which readily exhibit the effect of the powder cosmetics of the present invention include those containing a powder component and an oily component, for example, powder foundation, eye shadow, cheek color, face powder. Among these cosmetics, press-packed cosmetics are preferred, and particularly powder foundation and eye shadow are preferred. When the powder cosmetics of the present invention are formulated into powder foundation and eye shadow, the products are excellent in moisture feeling and adhesion to the skin without dusty feeling, resulting in a long lasting make-up which naturally blends with the skin without unevenness, kink, somber color, nor coming-off.

### Examples

The present invention will be explained by the following Examples which are not intended as a limitation thereof.

### Reference Example 1

### Preparation of oblate cellulose particles (1):

Purified cellulose powder derived from wood pulp (Nippon Paper Chemicals Co., Ltd.; KC Flock W-400; moisture content 7% in an air dry state) was dried at 40°C under reduced pressure to remove the adsorbed moisture well to 0.1% or lower. This cellulose powder (49 g) was added together with grinding zirconia balls (20 mm in diameter) into a tightly-closable grinding zirconia vessel (500 ml volume), and further stearic acid was added so as to be 2% to the cellulose powder.

Thereafter, the above grinding vessel was placed on a planetary ball mill (German Fritsch Co.; P-5 Type) and subjected to grinding in a cycle of grinding rotation for 10 minutes and intermission for 10 minutes; this cycle was repeated 72 times continuously. In this operation, the rotational frequency was set at 200 rpm (grinding energy: about 10G (gravitational acceleration)). The ground material was obtained as powder.

This powder was suspended into water in a flow cell, in which the average particle size (average value of the width and length on the apparatus) was obtained using a particle size distribution measuring apparatus of laser diffraction scattering system (Seishin Enterprise Co., Ltd.; LMS-24). In this operation, the values of particle size in 50% of the integrated volume were used as the average particle size.

In the practical measurement of the particle size distribution, the resulting powder (50 mg) was dispersed in 10 ml of distilled water to give a suspension, which was added by drops into a sample-circulating bath containing water as a medium on the particle size distribution measuring apparatus to reach an appropriate concentration; then, the particle size distribution was measured. As a result, the average particle size of the powder was 16µm.

The average thickness of the resulting powder was obtained by observing particles directly under a scanning electron microscope (Hitachi High-Technologies Corporation; S-3400N), choosing plural particles equivalent to the average particle size obtained as mentioned above, measuring their thickness and averaging the resulting values.

Observation under a scanning electron microscope was carried out as follows. A trace amount of the resulting powder was placed on a sample stage of a scanning electron microscope, dried under reduced pressure, and subjected to vapor deposition with gold, platinum to give a microscopic sample. The sample was observed at an accelerating voltage of 20 - 25 kV and a magnification of 500 - 10,000 to give an image, from which plural particles equivalent to the average particle size obtained as mentioned above were chosen. Further, their thickness was measured and the average thickness was obtained therefrom. As a result, the average thickness was 3µm.

Thus resulting powder was oblate cellulose particles having the average particle size of 16µm, the average thickness of 3µm, and the oblateness (average particle size/average thickness) of 5.3.

For comparison, the average particle sizes and the average thicknesses of the purified cellulose powder derived from wood pulp as raw material for the oblate cellulose particles (Nippon Paper Chemicals Co., Ltd.; KC Flock W-400) (hereinafter merely referred to as "cellulose powder") and of hemp cellulose powder (Tosco Co., Ltd.; Tosco Hemp Cellulose Powder) were measured in order to obtain the oblatenesses (average particle size/average thickness). The cellulose powder was not in oblate shape but in the shape of firewood; in this situation, a tentative measurement indicated that the average particle size was 28µm, the average thickness was 10µm, and the oblateness was 2.8. On the other hand, the hemp cellulose powder was not in oblate shape nor in firewood shape, but in granular shape; a tentative measurement indicated that the average particle size was 7.7µm, but the average thickness and oblateness could not be determined.

### Test Example 1

### Comparison of oil absorption

### (1) Measurement of oil absorption

The oil absorption on the oblate cellulose particles obtained in Reference Example 1 was measured according to JIS K 5101 "Method for Measurement of Oil Absorption". For comparison, the oil absorption of cellulose powder was measured in the same way.

The amount of the absorbed oil was 120 ml/100 g in the oblate cellulose particles obtained in Reference Example 1, and was 167.5 ml/100g in the cellulose powder.

### Example 1

### Comparison among products solidified with oil (1):

According to the prescription as shown in Table 1, the oblate cellulose particles prepared in Reference Example 1, cellulose powder, hemp cellulose powder (Tosco Co., Ltd.; Tosco Hemp Cellulose Powder), talc or sericite was homogeneously mixed with an oily component (squalane) and press-packed in a metallic mold to give solid product. The surface state of this product was evaluated visually, and the ease of removal with a sponge was evaluated according to the following evaluation standard. The results are shown in Table 1.

**[Table 1]**

| Component (%) | Product 1 | Comparative product 1 | Product 2 | Comparative product 2 | Comparative product 3 | Comparative product 4 | Comparative product 5 | Product 3 | Product 4 | Comparative product 6 | Comparative product 7 | Comparative product 8 | Comparative product .9 |
|---|---|---|---|---|---|---|---|---|---|---|---|---|---|
| Oblate cellulose particles ¹⁾ | 95 | - | 93 | - | - | - | - | 82 | 80 | - | - | - | - |
| Cellulose powder ²⁾ | - | - | - | 93 | - | - | - | - | - | 80 | - | - | - |
| Hemp Cellulose powder ³⁾ | - | - | - | - | 93 | - | - | - | - | - | 80 | - | - |
| Talc | - | 95 | - | - | - | 93 | - | - | - | - | - | 80 | - |
| Sericite | - | - | - | - | - | - | 93 | - | - | - | - | - | 80 |
| Squalane | 5 | 5 | 7 | 7 | 7 | 7 | 7 | 18 | 20 | 20 | 20 | 20 | 20 |
| State of surface | Powdery | Powdery | Powdery | Dry | Powdery | Firm | Firm | Powdery | Powdery | Dry | Firm | Glassy hard | Glassy hard |
| Ease of removal with a sponge | ○ | ○ | ○ | □ | ○ | × | Δ | ○ | ○ | □ | Δ | × | × |

| | | | | | | | | | | | | | |
|---|---|---|---|---|---|---|---|---|---|---|---|---|---|
| 1) Prepared in Reference Example 1 2) KC Flock W-400 (Nippon Paper Chemicals Co., Ltd.) 3) Tosco Hemp Cellulose Powder (Tosco Co., Ltd.) | | | | | | | | | | | | | |

### <Evaluation standard for the ease of removal with a sponge>

### Evaluation: Details

○: Removed well (No caking occurs even after repeated removal with a sponge)
×: Not removed (Caking occurs after rubbing 3 - 4 times with a sponge and cannot be removed)
Δ: Removed with difficulty (Removal is hindered by its firmness though not to such an extent as in talc)
D: Excessively removed (Excessively removed because of dried-out state)

### <Results>

In the product prepared by solidifying the oblate cellulose with the oil, the surface was kept in a powdery state even in blending of 5% or 7% oil and removed well with a sponge (Products 1 and 3). Further, even when the content of the oil was 18% or 20%, the surface was still in a powdery state and removed well with a sponge (Products 3 and 4) .

On the other hand, the product using talc solidified with the oil was also in a powdery state and removed well with a sponge when the content of the oil was 5%, but when the content reached 7%, the surface became firm and could not be removed in the least with a sponge (Comparative products 1 and 4). When the content of the oil was increased to 20%, the surface became like a glassy hard stone and could not be removed in the least with a sponge (Comparative product 8).

The product using sericite solidified with the oil had a firm surface even though the content of the oil was only 7% and could not be removed in the least with a sponge (Comparative product 5). When the content of the oil was up to 20%, the surface became like a glassy hard stone and could not be removed in the least with a sponge (Comparative product 9).

The product using cellulose powder solidified with the oil had a dried-out surface and was excessively removed with a sponge when the content of the oil was only 7% (Comparative product 2). Even though the content of the oil was 20%, the surface was still in a dried-out state and excessively removed with a sponge, though hardened in some degree with the sponge (Comparative product 6).

In the product using hemp cellulose solidified with the oil, there is no problem in the surface state and the removal with a sponge when the content of the oil was 7% (Comparative product 3). However, when the content of the oil reached 20%, the surface became hard and was difficult to remove with a sponge (Comparative product 7).

Thus, only the powder foundation prepared from oblate cellulose particles showed excellent properties in a wide range of the amount of the oily component without causing any trouble in the state of surface and the removal with sponge.

### Example 2

### Preparation of powder foundations (1):

As shown in Table 2, powder foundations were prepared according to the following production process. These powder foundations were evaluated for the surface state and the ease of removal with a sponge according to the same evaluation standard as in Example 1. The results are shown in Table 2.

**[Table 2]**

| Component (%) | | Product 5 | Comparative product 10 | Product 6 | Product 7 | Comparative product 11 | Comparative product 12 | Comparative product 13 | Comparative product 14 |
|---|---|---|---|---|---|---|---|---|---|
| 1 | Oblate cellulose particles¹⁾ | 51 | - | 10 | 30 | - | - | - | - |
| 2 | Talc | - | 51 | 50 | 27 | 63 | 61 | - | - |
| 3 | Sericite | - | - | - | - | - | - | 60 | 59 |
| 4 | Titanium dioxide | 10 | 10 | 10 | 10 | 10 | 10 | 10 | 10 |
| 5 | Mica | 15 | 15 | 15 | 15 | 15 | 15 | 15 | 15 |
| 6 | Iron oxide yellow | 1.5 | 1.5 | 1.5 | 1.5 | 1.5 | 1.5 | 1.5 | 1.5 |
| 7 | Iron oxide red | 0.4 | 0.4 | 0.4 | 0.4 | 0.4 | 0.4 | 0.4 | 0.4 |
| 8 | Iron black | 0.1 | 0.1 | 0.1 | 0.1 | 0.1 | 0.1 | 0.1 | 0.1 |
| 9 | Ester oil²⁾ | 11 | 11 | 6.5 | 8 | 5 | 6 | 6.5 | 7 |
| 10 | Silicone oil³⁾ | 11 | 11 | 6.5 | 8 | 5 | 6 | 6.5 | 7 |
| State of surface | | Powdery | Glassy hard | Powdery | Powdery | Powdery | Hard | Powdery | Hard |
| Ease of removal with a sponge | | ○ | × | ○ | ○ | ○ | × | ○ | × |

| | | | | | | | | | |
|---|---|---|---|---|---|---|---|---|---|
| 1) Prepared in Reference Example 1 2) OOD (Shin-Ei Chemical Co., Ltd.) 3) KF99-1 (Shin-Etsu Chemical Co., Ltd.) | | | | | | | | | |

### <Production process>

A: Components (1) to (8) are mixed and dispersed homogeneously.
B: Components (9) and (10) are mixed homogeneously at room temperature.
C: B is dispersed into A.
D: C is press-packed in a metallic mold to give a powder foundation.

### <Results>

The powder foundation prepared with the oblate cellulose particles and the oily components (ester oil, silicone oil) had a powdery surface, which could be removed with a sponge without caking at all (Product 5). On the other hand, in the powder foundation prepared with talc and the oily components in the same ratio, the surface was in a glassy and hard state and could not be removed in the least with a sponge (Comparative product 10).

The powder foundations containing the oblate cellulose particles had no problem in the property even when the content of the oily components was 13 - 22% (Products 5 - 7).

On the other hand, there was no problem in the powder foundation containing talc together with 10% oily components (Comparative product 11), but when the content of the oily components was 12% (Comparative product 12), caking occurred.

In addition, there was no problem in the powder foundation containing sericite together with 13% oily components (Comparative product 13), but when the content of the oily components was 14% (Comparative product 14), caking occurred.

In this situation, a combination of talc and oblate cellulose particles made it possible to increase the amount of the oily components to be compounded, in comparison with the use of talc alone (Products 6 and 7).

### Example 3

### Preparation of powder foundations (2):

### (1) Preparation of powder foundation

As shown in Table 3, a powder foundation was prepared according to the following production process. The powder foundation was evaluated for the surface state and the ease of removal with a sponge according to the same evaluation standard as in Example 1. The results are shown in Table 3.

**[Table 3]**

| Component (Part by weight) | | Product 8 |
|---|---|---|
| 1 | Oblate cellulose particels¹⁾ | 33 |
| 2 | Titanium dioxide | 10 |
| 3 | Mica | 15 |
| 4 | Iron oxide yellow | 1.5 |
| 5 | Iron oxide red | 0.4 |
| 6 | Iron black | 0.1 |
| 7 | Ester oil²⁾ | 10 |
| 8 | Silicone oil³⁾ | 10 |
| State of surface | | Powdery |
| Ease of removal with a sponge | | ○ |

| | | |
|---|---|---|
| 1)Prepared in Reference Example 1 2) OOD (Shin-Ei Chemical Co., Ltd.) 3) KF99-1 (Shin-Etsu Chemical Co., Ltd.) | | |

### <Production process>

A: Components (1) to (6) are mixed and dispersed homogeneously.
B: Components (7) and (8) are mixed homogeneously at room temperature.
C: B is dispersed into A.
D: C is press-packed in a metallic mold to give 8g of powder foundation.

### <Results>

The powder foundation prepared as described above had a powdery surface and could be removed with a sponge without causing any problem.

### (2) Preparation of powder foundation in which the content of oily components is increased

In the powder foundation (8 g) prepared in the above item (1), the content of oily components was increased from 20 parts by weight (25%) to 40 parts by weight (40%) according to the following process as described in JP 7/55892B (1995) (Product 9).

### <Process for increasing the content of oily components>

E: Onto the powder foundation were added 2 g of oily components (a 1:1 mixture of the components (7) and (8)) without damaging the surface of the powder foundation so that it was absorbed into the powder.

### <Results>

There was no change in the state of the surface of the above-prepared powder foundation in which the content of oily components was increased as compared with that before addition of the oily components, and no oozing-out of the oily components was observed. In addition, there was no problem in the removal with a sponge, and no caking occurred on the surface.

In the powder foundation prepared according to the process as described in JP 7/55892B (1995), it was necessary to make holes on the metallic plate in order to let off the excess oily component that the powder was unable to absorb. On the other hand, the powder foundation of the present invention requires no hole on the metallic plate, since it has a high absorbability for the oily component owing to the use of the oblate cellulose particles.

### Example 4

### Preparation of eye shadows:

As shown in Table 4, eye shadows were prepared according to the following production process. These eye shadows were evaluated for the ease of removal with a sponge according to the same evaluation standard as in Example 1. The results are shown in Table 4.

**[Table 4]**

| Component (%) | | Product 10 | Product 11 | Comparative product 15 | Comparative product 16 |
|---|---|---|---|---|---|
| 1 | Oblate cellulose particles ¹⁾ | 38.0 | 33.0 | - | - |
| 2 | Mica | 20.0 | 20.0 | 20.0 | 20.0 |
| 3 | Talc | - | - | 47.0 | 43.0 |
| 4 | Iron oxide yellow | 4.0 | 4.0 | 4.0 | 4.0 |
| 5 | Ultramarine | 3.0 | 3.0 | 3.0 | 3.0 |
| 6 | Pearle pigment ²⁾ | 10.0 | 10.0 | 10.0 | 10.0 |
| 7 | Ester oil³⁾ | 13.0 | 15.0 | 8.0 | 10.0 |
| 8 | Silicone oil⁴⁾ | 12.0 | 15.0 | 8.0 | 10.0 |
| Ease of removal with a sponge | | ○ | ○ | ○ | × |

| | | | | | |
|---|---|---|---|---|---|
| 1) Prepared in Reference Example 1 2) Prominence SP (Nihon Koken Kogyo Co., Ltd.) 3) OOD (Shin-Ei Chemical Co., Ltd.) 4) KF99-1 (Shin-Etsu Chemical Co., Ltd.) | | | | | |

### <Production process>

A: Components (1) to (6) are mixed and dispersed homogeneously.
B: Components (7) and (8) are mixed homogeneously at room temperature.
C: B is mixed and dispersed into A.
D: C is press-packed in a metallic mold to give an eye shadow.

### <Results>

In the eye shadow prepared from the oblate cellulose particles and the oily components, there was no problem in the removal with a sponge even when the content of the oily components was 25% or 30% (Products 10 and 11).

On the other hand, in the eye shadow prepared from mica, talc and the oily components, there is no problem in the removal with a sponge when the content of the oily components was 16% (Comparative product 15), but a problem occurred in the removal with a sponge when the content was 20% (Comparative product 16).

### Reference Example 2

### Preparation of oblate cellulose particles (2):

Purified cellulose powder derived from wood pulp (Nippon Paper Chemicals Co., Ltd.; KC Flock W-400; moisture content 7% in an air dry state) was dried at 40°C under reduced pressure to remove the adsorbed moisture well to 0.1% or lower. This cellulose powder (49 g) was added together with grinding zirconia balls (20 mm in diameter) into a tightly-closable grinding zirconia vessel (500 ml volume), and further stearic acid was added so as to be 2%, and triethoxysilylethylpolydimethylsiloxyethyldimethicone (Shin-Etsu Chemical Co., Ltd.) so as to be 1% to the cellulose powder.

Thereafter, the above grinding vessel was placed on a planetary ball mill (German Fritsch Co.; P-5 Type) and subjected to grinding in a cycle of grinding rotation for 10 minutes and intermission for 10 minutes; this cycle was repeated 72 times continuously. In this operation, the rotational frequency was set at 200 rpm (grinding energy: about 10G (gravitational acceleration)). The ground material was obtained as powder. The average particle size, average thickness and oblateness were measured in the same manner as in Reference Example 1. As a result, the average particle size was 16µm, the average thickness was 2µm, and the oblateness was 8.

### Reference Example 3

### Preparation of oblate cellulose particles (3):

Purified cellulose powder derived from wood pulp (Nippon Paper Chemicals Co., Ltd.; KC Flock W-400; moisture content 7% in an air dry state) was dried at 40°C under reduced pressure to remove the adsorbed moisture well to 0.1% or lower. This cellulose powder (49 g) was added together with grinding zirconia balls (20 mm in diameter) into a tightly-closable grinding zirconia vessel (500 ml volume), and further pure water was added so as to be 3%, and N^{ε}-lauroyl-L-lysine (Amihope®LL; Ajinomoto Co., Ltd.) so as to be 2% to the cellulose powder.

Thereafter, the above grinding vessel was placed on a planetary ball mill (German Fritsch Co.; P-5 Type) and subjected to grinding in a cycle of grinding rotation for 10 minutes and intermission for 10 minutes; this cycle was repeated 72 times continuously. In this operation, the rotational frequency was set at 200 rpm (grinding energy: about 10G (gravitational acceleration)). The ground material was obtained as powder. The average particle size, average thickness and oblateness were measured in the same manner as in Reference Example 1. As a resut, the average particle size was 21µm, the average thickness was 0.3µm, and the oblateness was 70. This powder had the lower average thickness and the higher oblateness than those prepared in Reference Examples 1 and 2.

### Test Example 2

### Comparison of hydrophobicity and oil absorption

### (1) Hydrophobicity of oblate cellulose particles

The respective oblate cellulose particles (about 0.1 g) prepared in Reference Examples 1 to 3 were added to 50 ml of water in a glass vessel, and agitated with a glass stick around 50 times. The respective states after agitation were observed visually to determine the hydrophobicity of the oblate cellulose particles.

When the oblate cellulose particles prepared in Reference Example 1 was dispersed in water, the water became clouded, indicating no hydrophobicity. On the other hand, the respective oblate cellulose particles prepared in Reference Examples 2 and 3 floated on the water surface without being dispersed, indicating their positive hydrophobicity.

### (2) Hydrophobicity of mixtures

An oil (squalane)(2g) and the respective oblate cellulose particles (8g) prepared in Reference Examples 1 to 3 were agitated with a mixer for around 15 seconds to give homogeneously mixed mixtures 1 to 3. These mixtures (about 0.1g each) were taken and agitated with a glass stick around 50 times. The respective states after agitation were observed visually to determine the hydrophobicity of the powders .

When the mixture 1 containing the oblate cellulose particles prepared in Reference Example 1 was dispersed in water, the water became clouded, indicating no hydrophobicity. On the other hand, the mixtures 2 and 3 containing the oblate cellulose particles prepared in Reference Examples 2 and 3, respectively, remained floating on the water surface after standing for a whole day and night, indicating their positive hydrophobicity.

### (3) Measurement of contact angle

The respective mixtures prepared in the above item (2) were placed on a metallic mold and pressed with a press machine to make the surface even. A drop of water was put on the surface, and the droplet on the powder was photographed for purpose of measuring its contact angle by a circular protractor.

The contact angle was 64° in the mixture 1 containing the oblate cellulose particles prepared in Reference Example 1; 105° in the the mixture 2 containing the oblate cellulose particles prepared in Reference Example 2; and 117° in the mixture 3 containing the oblate cellulose particles prepared in Reference Example 3.

### (4) Measurement of oil absorption

The oil absorption on the respective oblate cellulose particles prepared in Reference Examples 1 to 3 was measured according to the method of JIS K 5101 for measuring oil absorption.

The amount of the absorbed oil was 134 ml/100 g in the oblate cellulose particles prepared in Reference Example 1; 123 ml/100 g in the oblate cellulose particles prepared in Reference Example 2; and 139 ml/100 g in the oblate cellulose particles prepared in Reference Example 3. From these results, it was found that the treatment of the oblate cellulose particles with a hydrophobizing agent produces no change in the property of the mixture of the oblate cellulose particles and the oil.

### Example 5

### Comparison among products solidified with oil (2):

As shown in Table 5, solid products were prepared using the respective oblate cellulose particles prepared in Reference Examples 1 to 3 according to the same production process as in Example 1. The surface of these products was evaluated visually, and the removal with a sponge and their extension on the skin were evaluated freely. For comparison, a solid product was prepared in the same way from the cellulose powder as raw material for the oblate cellulose particles and evaluated similarly. The results are shown in Table 5.

**[Table 5]**

| Component (%) | | Product 12 | Product 13 | Product 14 | Comparatice product 17 |
|---|---|---|---|---|---|
| 1 | Oblate cellulose particles¹⁾ | 80 | - | - | - |
| 2 | Oblate cellulose particles²⁾ | - | 80 | - | - |
| 3 | Oblate cellulose particles³⁾ | - | - | 80 | - |
| 4 | Powder cellulose particles⁴⁾ | - | - | - | 80 |
| 5 | Squalane | 20 | 20 | 20 | 20 |
| State of surface | | Solid | Rather hard | Firmly pressed with elasticity | Fragile and easily collapsed |
| Ease of removal with a sponge | | Normal | Smaller than in Product 12 | Removed thicker than in Product 12 at a time | Removed in a block form |
| Extension on skin | | Good | Smooth | Extensible evenly | Worse |
| Result of evaluation | | Suitable as powder ingredient | Suitable as powder ingredient | Greatly superior as powder ingredient | Unsuitable as powder ingredient |

| | | | | | |
|---|---|---|---|---|---|
| 1) Prepared in Reference Example 1 2) Prepared in Reference Example 2 3) Prepared in Reference Example 3 4) KC Flock W-400 (Nippon Paper Chemicals Co., Ltd.) | | | | | |

### <Results>

The product using the oblate cellulose particles prepared in Reference Example 1 and solidified by the oil, had a powdery surface, which could be removed with a sponge in the 2nd-ranked amount among the Products 12 - 14. Spread on the skin was highly satisfactory (Product 12).

The product using the oblate cellulose particles prepared in Reference Example 2 and solidified by the oil, had a slightly hard and firm surface, which did not cause caking, and could be smoothly spread on the skin (Product 13).

Further, the product using the oblate cellulose particles prepared in Reference Example 3 and solidified by the oil, had a firm surface, which could be removed with a sponge in the 1st-ranked amount among the three products, and could be spread evenly on the skin (Product 14).

On the other hand, the product using powder cellulose particles solidified by the oil was wiped away in a block form and its spread on the skin was worse (Comparative product 17).

### Example 6

### Preparation of powder foundations (3):

As shown in Table 6, powder foundations were prepared according to the following production process. These powder foundations were tried and scored by 9 make-up specialists as panelists in terms of removal with a sponge, the state of the cake surface after wiping-away, adhesiveness to the skin (spread, texture, touch), and make-up durability (water-resisting) according to the following score criterion, and the average marks were evaluated according to the following evaluation standard. The results are shown in Table 6.

**[Table 6]**

| Composition (%) | | Comparative product 18 | Product 15 | Product 16 | Product 17 | Comparative product 19 | Product 18 | Product 19 | Product 20 |
|---|---|---|---|---|---|---|---|---|---|
| 1 | Powder cellulose particle ¹⁾ | 35.0 | - | - | - | 25.0 | - | - | - |
| 2 | Oblate cellulose particle ²⁾ | - | 35.0 | - | - | - | 25.0 | - | - |
| 3 | Oblate cellulose particle.³⁾ | - | - | 35.0 | - | - | - | 25.0 | - |
| 4 | Oblate cellulose particle⁴⁾ | - | - | - | 35.0 | - | - | - | 25.0 |
| 5 | Titanium dioxide | 18.0 | 18.0 | 18.0 | 18.0 | 18.0 | 18.0 | 18.0 | 18.0 |
| 6 | Mica | r. a. | r. a. | r. a. | r. a. | r. a. | r. a. | r. a. | r. a. |
| 7 | Nylon powder⁵⁾ | 15.0 | 15.0 | 15.0 | 15.0 | 15.0 | 15.0 | 15.0 | 15.0 |
| 8 | Iron oxide yellow | 1.34 | 1.34 | 1.34 | 1.34 | 1.34 | 1.34 | 1.34 | 1.34 |
| 9 | Iron oxide red | 0.53 | 0.53 | 0.53 | 0.53 | 0.53 | 0.53 | 0.53 | 0.53 |
| 10 | Iron black | 0.13 | 0.13 | 0.13 | 0.13 | 0.13 | 0.13 | 0.13 | 0.13 |
| 11 | Ester oil⁶⁾ | 1.5 | 1.5 | 1.5 | 1.5 | 3.0 | 3.0 | 3.0 | 3.0 |
| 12 | Squalane | 1.5 | 1.5 | 1.5 | 1.5 | 3.0 | 3.0 | 3.0 | 3.0 |
| 13 | Liquid paraffin | 3.0 | 3.0 | 3.0 | 3.0 | 6.0 | 6.0 | 6.0 | 6.0 |
| 14 | Silicone oil⁷⁾ | 3.0 | 3.0 | 3.0 | 3.0 | 6.0 | 6.0 | 6.0 | 6.0 |
| 15 | Silicone oil⁸⁾ | 3.0 | 3.0 | 3.0 | 3.0 | 6.0 | 6.0 | 6.0 | 6.0 |
| 16 | Ceresin wax | 0.5 | 0.5 | 0.5 | 0.5 | 1.0 | 1.0 | 1.0 | 1.0 |
| 17 | Antiseptic | a. a. | a. a. | a. a. | a. a. | a. a. | a. a. | a. a. | a. a. |
| 18 | Perfume | a. a. | a. a. | a. a. | a. a. | a. a. | a. a. | a. a. | a. a. |
| Removal with a sponge | | Δ | ○ | ○ | ○ | Δ | ⊙ | ⊙ | ⊙ |
| State of surface | | Δ | ○ | ○ | ○ | Δ | ⊙ | ⊙ | ⊙ |
| Adhesiveness to skin | | Δ | ○ | ○ | ○ | Δ | ⊙ | ⊙ | ⊙ |
| Evenness of make-up film | | ○ | ○ | ○ | ○ | ○ | ⊙ | ⊙ | ⊙ |
| Make-up durability | | Δ | ○ | ○ | ○ | ○ | ⊙ | ⊙ | ⊙ |

| | | | | | | | | | |
|---|---|---|---|---|---|---|---|---|---|
| Note: r. a.= residual amount; a. a.= adequate amount 1) KC Flock W-400 (Nippon Paper Chemicals Co., Ltd.) 2) Prepared in Reference Example 1 3) Prepared in Reference Example 2 4) Prepared in Reference Example 3 5) Orgasol 2002 EXD NAT COS (ARKEMA CO.) 6) OOD (Shin-Ei Chemical Co., Ltd.) 7) KF-99-1 (Shin-Etsu Chemical Co., Ltd.) 8) KF-56 (Shin-Etsu Chemical Co., Ltd.) | | | | | | | | | |

### <Production process>

A: Components (1) to (10) are mixed and dispersed homogeneously.
B: Components (11) to (17) are mixed homogeneously under heating at 70°C.
C: B kept at 50°C is mixed with Component (18) homogeneously.
D: A is mixed and dispersed into C homogeneously.
E: D is press-packed in a metallic plate to give a powder foundation.

### <Score criteria>

### Score: Details

5: Excellent
4: Good
3: Fair
2: Weak
1: Poor

### <Comprehensive evaluation>

### Evaluation: Average score

⊙: Score 5 or less to 4 or more
○: Score less than 4 to 3 or more
Δ: Score less than 3 to 2 or more
× : Score less than 2 to 1 or more

### <Results>

The evaluation showed that there is a problem in touch (texture and dried-out feeling) when the powder cellulose is used as extender.

In contrast, the three members of oblate cellulose prepared in Reference Examples received high scores in the sense of use and the aspect of make-up film, indicating their excellent usability as extender.

### Example 7

### Preparation of powder foundations (4):

As shown in Table 7, powder foundations were prepared according to the following production process. These powder foundations were tried and evaluated freely by 9 make-up specialists as panelists for the aspect of make-up film. The results are also shown in Table 7.

**[Table 7]**

| Component (%) | | Comparative product 20 | Comparative product 21 | Product 21 | Comparative product 22 | Comparative product 23 | Product 22 |
|---|---|---|---|---|---|---|---|
| 1 | Titanium dioxide | 12 | 12 | 12 | 18 | 18 | 18 |
| 2 | Mica | r. a. | r. a. | r. a. | r. a. | r. a. | r. a. |
| 3 | Nylon powder¹⁾ | 15 | 15 | 15 | 15 | 15 | 15 |
| 4 | Talc | 40 | - | - | 40 | - | - |
| 5 | Sericite | - | 40 | - | - | 40 | - |
| 6 | Oblate cellulose²⁾ | - | - | 40 | - | - | 40 |
| 7 | Iron oxide yellow | 1.34 | 1.34 | 1.34 | 1.34 | 1.34 | 1.34 |
| 8 | Iron oxide red | 0.53 | 0.53 | 0.53 | 0.53 | 0.53 | 0.53 |
| 9 | Iron black | 0.13 | 0.13 | 0.13 | 0.13 | 0.13 | 0.13 |
| 10 | Ester oil³⁾ | 1.5 | 1.5 | 1.5 | 1.5 | 1.5 | 1.5 |
| 11 | Squalane | 1.5 | 1.5 | 1.5 | 1.5 | 1.5 | 1.5 |
| 12 | Liquid paraffin | 3 | 3 | 3 | 3 | 3 | 3 |
| 13 | Silicone oil⁴⁾ | 3 | 3 | 3 | 3 | 3 | 3 |
| 14 | Silicone oil⁵⁾ | 3 | 3 | 3 | 3 | 3 | 3 |
| 15 | Ceresin wax | 0.5 | 0.5 | 0.5 | 0.5 | 0.5 | 0.5 |
| 16 | Antiseptic | a. a. | a. a. | a. a. | a. a. | a. a. | a. a. |
| 17 | Perfume | a. a. | a. a. | a. a. | a. a. | a. a. | a. a. |
| Aspect of make-up film | | Adequate transparency and make-up effect | Adequate whiteness and make-up effect | Transparency and light make-up | Opaque and heavy make-up | Unnatural whiteness and heavy make-up | Adequate make-up effect and natural make-up film |

| | | | | | | | |
|---|---|---|---|---|---|---|---|
| 1) Orgasol 2002 EXD NAT COS (ARKEMA Co.) 2) Prepared in Reference Example 3 3) OOD (Shin-Ei Chemical Co., Ltd.) 4) KF-99-1 (Shin-Etsu Chemical Co., Ltd.) 5) KF-56 (Shin-Etsu Chemical Co., Ltd.) | | | | | | | |

### <Production process>

A: Components (1) to (9) are mixed and dispersed homogeneously.
B: Components (10) to (16) are mixed homogeneously under heating at 70°C.
C: B kept at 50°C is mixed with Component (17) homogeneously.
D: A is mixed and dispersed into C homogeneously.
E: D is press-packed in a metallic plate to give a powder foundation.

### <Results>

As a result, it was found that there is no great difference in the make-up effect among the three raw materials (talc, sericite, oblate cellulose) when the titanium dioxide content is up to the level of 12%, but when the content is raised up to 18%, the finishing of make-up becomes clearly unnatural by the use of talc or sericite, while an adequate make-up effect and the natural make-up film can be kept by the use of oblate cellulose.

### Example 8

### Preparation of powder foundations (5):

As shown in Table 8, powder foundations were prepared according to the following production process. These powder foundations were tried and evaluated freely by 9 make-up specialists as panelists for the aspect of make-up film. The results are also shown in Table 8.

**[Table 8]**

| Component (%) | | Product 23 | Product 24 | Product 25 |
|---|---|---|---|---|
| 1 | Titanium dioxide | 18.0 | 21.0 | 24.0 |
| 2 | Mica | r. a. | r. a. | r. a. |
| 3 | Nylon powder¹⁾ | 15.0 | 15.0 | 15.0 |
| 4 | Oblate cellulose²⁾ | 30.0 | 30.0 | 30.0 |
| 5 | Iron oxide yellow | 1.34 | 1.34 | 1.34 |
| 6 | Iron oxide red | 0.53 | 0.53 | 0.53 |
| 7 | Iron black | 0.13 | 0.13 | 0.13 |
| 8 | Ester oil³⁾ | 3.0 | 3.0 | 3.0 |
| 9 | Squalane | 3.0 | 3.0 | 3.0 |
| 10 | Liquid paraffin | 6.0 | 6.0 | 6.0 |
| 11 | Silicone oil⁴⁾ | 6.0 | 6.0 | 6.0 |
| 12 | Silicone oil⁵⁾ | 6.0 | 6.0 | 6.0 |
| 13 | Ceresin wax | 1.0 | 1.0 | 1.0 |
| 14 | Antiseptic | a. a. | a. a. | a. a. |
| 15 | Perfume | a. a. | a. a. | a. a. |
| Aspect of make-up film | | Adequate transparency and make-up effect | Adequate whiteness and natural make-up film | Firm make-up film and quite covering make-up film |

| | | | | |
|---|---|---|---|---|
| 1) Orgasol 2002 EXD NAT COS (ARKEMA Co.) 2) Prepared in Reference Example 3 3) OOD (Shin-Ei Chemical Co., Ltd.) 4) KF-99-1 (Shin-Etsu Chemical Co., Ltd.) 5) KF-56 (Shin-Etsu Chemical Co., Ltd.) | | | | |

### <Production process>

A: Components (1) to (7) are mixed and dispersed homogeneously.
B: Components (8) to (14) are mixed homogeneously under heating at 70°C.
C: B kept at 50°C is mixed with Component (15) homogeneously.
D: A is mixed and dispersed into C homogeneously.
E: D is press-packed in a metallic plate to give a powder foundation.

### <Results>

It was found in this experiment that oblate cellulose, particularly the oblate cellulose prepared in Reference Example 3 (grinding aid: water; hydrophobizing agent: N^{ε}-lauroyl-L-lysine), has a very high oblateness, and is capable of achieving the effect which is unattainable in using the conventional extender. In the conventional powder foundations prepared based on talc or sericite, the content of titanium dioxide to be added is limited to about 12%. The content over this line results in unnatural finishing, and extension on the skin shows a tendency to become heavy. On the other hand, since the lauroyllysine-processed oblate cellulose is thin and *per se* highly transparent, it is possible to admix titanium dioxide up to 24% without accompanying unnatural whiteness and any problem in extensibility on the skin; to the contrary, increase of the amount of titanium dioxide increases a covering effect to yield a fine make-up film.

### Industrial Applicability

The amount of oily components which can be added to the conventional powder cosmetics containing talc or sericite as extender is limited to about 15% at the most, and when it was added over this level, caking occurred to worsen the ease of removal with a sponge, resulting in making it inappropriate practically. On the other hand, the powder cosmetic of the present invention in which oblate cellulose particles are used as extender, can contain a large amount of oily components without sacrificing the powdery feel of the product.

In the conventional powder cosmetics, the amount of titanium dioxide to be compounded was limited because it causes unnatural whiteness and thickness when applied. To the contrary, in the powder cosmetics containing oblate cellulose particles as extender, the unnatural whiteness and thickness do not appear when applied, even though a large amount of titanium dioxide is added.

According to the present invention, it is possible to prepare powder cosmetics in which the contents of oily components and titanium dioxide are increased, depending on the combination of powders.

### Brief Description of Drawings

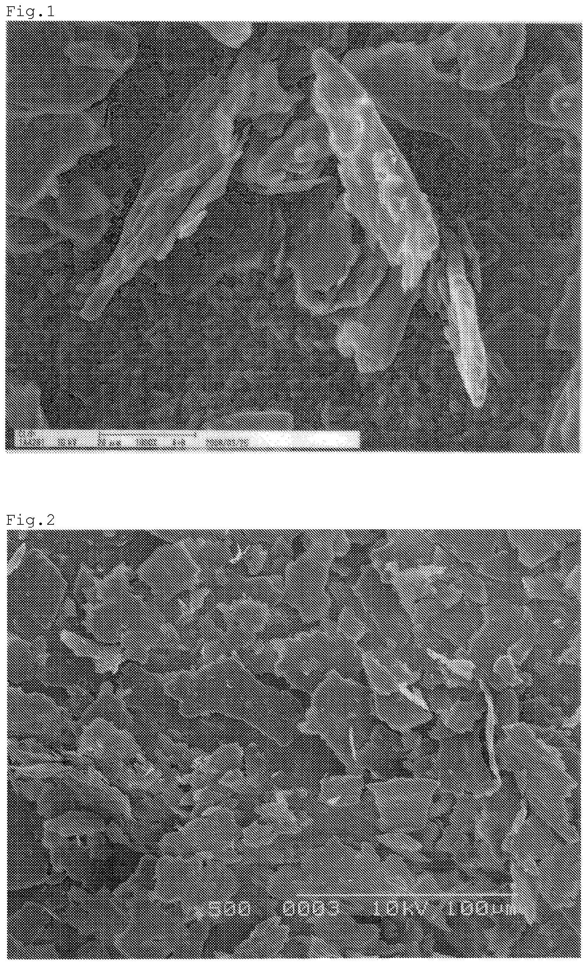

Fig. 1 shows an electron microphotograph (1, 000 magnifications) of the oblate cellulose particles prepared in Reference Example 1.
Fig.2 shows an electron microphotograph (500 magnifications) of the oblate cellulose particles prepared in Reference Example 3.

## Claims

1. A powder cosmetic comprising oblate cellulose particles and an oily component, wherein the content of the oily component is 15 to 40% by mass.

2. The powder cosmetic according to claim 1, wherein the oblate cellulose particles are prepared by the following process (a) or (b):
(a) a process for mechanically grinding a mixture comprising a cellulosic material and at least one of grinding aids selected from the group consisting of water, fatty acids, synthetic polymers and organic solvents;
(b) a process for mechanically grinding a cellulosic material after frozen at a low temperature.

3. The powder cosmetic according to claim 2, wherein the mixture of the process (a) further comprises at least one of hydrophobizing agents selected from the group consisting of N-acyl amino acids and reactive silicones.

4. The powder cosmetic according to claim 2 or 3, wherein the cellulosic material is fibrous or powdered wood flour or wood pulp derived from wood, fibrous or powdered cotton or linter fiber derived from raw cotton, or their purified fibrous or powdered cellulosic materials.

5. The powder cosmetic according to any one of claims 1 to 4, further comprising 15 - 30% by mass of titanium dioxide.

6. The powder cosmetic according to any one of claims 1 to 5, wherein said powder cosmetic is a powder foundation.

7. The powder cosmetic according to any one of claims 1 to 5, wherein said powder cosmetic is an eye shadow.

8. The powder cosmetic according to any one of claims 1 to 5, wherein said powder cosmetic is a face powder.

9. Oblate cellulose particles which are prepared by mechanically grinding a mixture comprising a cellulosic material, an N-acyl amino acid, and at least one of grinding aids selected from the group consisting of water, fatty acids, synthetic polymers and organic solvents, wherein the average particle size is 1 - 100µm, the average thickness is 0.01 - 5µm, and the oblateness is 50 - 200.

10. A cosmetic comprising the oblate cellulose particles according to claim 9.

11. A method for producing oblate cellulose particles, comprising mechanically grinding a mixture comprising a cellulosic material, an N-acyl amino acid, and at least one of grinding aids selected from the group consisting of water, fatty acids, synthetic polymers and organic solvents, and obtaining oblate cellulose particles having the average particle size of 1 - 100µm, the average thickness of 0.01 - 5µm, and the oblateness of 50 - 200.

## Patentansprüche

1. Pulverförmiges Kosmetikprodukt mit abgeplatteten Cellulosepartikeln und mit einer öligen Komponente, wobei der Gehalt der öligen Komponente 15 bis 40 Masse-% beträgt.

2. Pulverförmiges Kosmetikprodukt nach Anspruch 1, wobei die abgeplatteten Cellulosepartikel durch das folgende Verfahren (a) oder (b) hergestellt werden:
(a) ein Verfahren zum mechanischen Mahlen einer Mischung, die ein cellulosisches Material und mindestens ein Mahlhilfsmittel enthält, die ausgewählt sind aus der Gruppe bestehend aus Wasser, Fettsäuren, synthetischen Polymeren und organischen Lösungsmitteln; und
(b) ein Verfahren zum mechanischen Mahlen eines cellulosischen Materials, nachdem es bei einer niedrigen Temperatur gefroren worden ist.

3. Pulverförmiges Kosmetikprodukt nach Anspruch 2, wobei die Mischung des Verfahrens (a) ferner mindestens ein Hydrophobisierungsmittel aufweist, die ausgewählt sind aus der Gruppe bestehend aus N-Acylaminosäuren und reaktionsfähigen Silikonen.

4. Pulverförmiges Kosmetikprodukt nach Anspruch 2 oder 3, wobei das cellulosische Material faseriges oder pulverisiertes Holzmehl oder Zellstoff, die aus Holz gewonnenen sind, faserige oder pulverisierte Baumwolle oder Linterfasern, die aus Rohbaumwolle gewonnen sind, oder deren gereinigte faserige oder pulverisierte cellulosische Materialien ist.

5. Pulverförmiges Kosmetikprodukt nach einem der Ansprüche 1 bis 4, ferner mit 15 bis 30 Masse-% Titandioxid.

6. Pulverförmiges Kosmetikprodukt nach einem der Ansprüche 1 bis 5, wobei das pulverförmige Kosmetikprodukt eine Pulvergrundierung ist.

7. Pulverförmiges Kosmetikprodukt nach einem der Ansprüche 1 bis 5, wobei das pulverförmige Kosmetikprodukt ein Lidschatten ist.

8. Pulverförmiges Kosmetikprodukt nach einem der Ansprüche 1 bis 5, wobei das pulverförmige Kosmetikprodukt ein Gesichtspuder ist.

9. Abgeplattete Cellulosepartikel, die durch mechanisches Mahlen einer Mischung hergestellt werden, die ein cellulosisches Material, eine N-Acylaminosäure und mindestens ein Mahlhilfsmittel aufweisen, die ausgewählt sind aus der Gruppe bestehend aus Wasser, Fettsäuren, synthetischen Polymeren und organischen Lösungsmitteln, wobei die mittlere Partikelgröße 1 bis 100 µm, die mittlere Dicke 0,01 bis 5 µm und die Abplattung 50 bis 200 betragen.

10. Kosmetikprodukt mit den abgeplatteten Cellulosepartikeln nach Anspruch 9.

11. Verfahren zum Herstellen abgeplatteter Cellulosepartikel, mit dem mechanischen Mahlen einer Mischung, die ein cellulosisches Material, eine N-Acylaminosäure und mindestens ein Mahlhilfsmittel aufweist, die ausgewählt sind aus der Gruppe bestehend aus Wasser, Fettsäuren, synthetischen Polymeren und organischen Lösungsmitteln, und Erhalten abgeplatteter Cellulosepartikel mit einer mittleren Partikelgröße von 1 bis 100 µm, einer mittleren Dicke von 0,01 bis 5 µm und einer Abplattung von 50 bis 200.

## Revendications

1. Cosmétique en poudre comprenant des particules de cellulose aplaties et un composant huileux, où la teneur du composant huileux est de 15 à 40 % en masse.

2. Cosmétique en poudre selon la revendication 1, dans lequel les particules de cellulose aplaties sont préparées par le procédé (a) ou le procédé (b) suivant :
(a) un procédé de mouture mécanique d'un mélange comprenant un matériau cellulosique et au moins une des aides pour la mouture choisie dans le groupe constitué de l'eau, des acides gras, des polymères synthétiques et de solvants organiques ;
(b) un procédé de mouture mécanique d'un matériau cellulosique après une congélation à basse température.

3. Cosmétique en poudre selon la revendication 2, dans lequel le mélange du procédé (a) comprend en outre au moins un des agents hydrophobes choisis dans le groupe constitué des acides N-acyl aminés et de silicones réactifs.

4. Cosmétique en poudre selon la revendication 2 ou 3, dans lequel le matériau cellulosique est de la farine de bois ou de la pulpe de bois fibreuse ou pulvérulente dérivée de bois, du coton ou de la fibre de duvet de coton fibreux ou pulvérulent provenant de coton brut, ou leurs matériaux cellulosiques purifiés fibreux ou pulvérulents.

5. Cosmétique en poudre selon l'une quelconque des revendications 1 à 4, comprenant en outre de 15 à 30 % en masse de dioxyde de titane.

6. Cosmétique en poudre selon l'une quelconque des revendications 1 à 5, où ledit cosmétique en poudre est un fond de teint en poudre.

7. Cosmétique en poudre selon l'une quelconque des revendications 1 à 5, où ledit cosmétique en poudre est une ombre à paupière.

8. Cosmétique en poudre selon l'une quelconque des revendications 1 à 5, où ledit cosmétique en poudre est une poudre pour le visage.

9. Particules de cellulose aplaties qui sont préparés par une mouture mécanique d'un mélange comprenant un matériau cellulosique, un acide N-acyl aminé, et au moins une des aides à la mouture choisie dans le groupe constitué de l'eau, des acides gras, des polymères synthétiques et de solvants organiques, où la taille particulaire moyenne est de 1 à 100 µm, l'épaisseur moyenne est de 0,01 à 5 µm, et l'aplatissement est de 50 à 200.

10. Cosmétique comprenant les particules de cellulose aplaties selon la revendication 9.

11. Procédé de production de particules de cellulose aplaties, comprenant une mouture mécanique d'un mélange comprenant un matériau cellulosique, un acide N-acyl aminé, et au moins une des aides à la mouture choisie dans le groupe constitué de l'eau, des acides gras, des polymères synthétiques et de solvants organiques, et l'obtention de particules de cellulose aplaties ayant la taille particulaire moyenne de 1 à 100 µM, l'épaisseur moyenne de 0,01 à 5 µm, et l'aplatissement de 50 à 200.
